(19) Europäisches Patentamt
European Patent Office
Office européen des brevets

(11) Numéro de publication: **0 116 494**
**B1**

# (12) FASCICULE DE BREVET EUROPEEN

(45) Date de publication du fascicule du brevet: **27.01.88**

(21) Numéro de dépôt: **84400157.8**

(22) Date de dépôt: **25.01.84**

(51) Int. Cl.⁴: **C 07 D 237/24**, C 07 D 409/04, C 07 D 403/04, C 07 D 413/12, A 61 K 31/50

## (54) Dérivés de la pyridazine ayant une action psychotrope, procédé d'obtention, intermédiaires et médicaments en contenant.

(30) Priorité: **28.01.83 FR 8301366**
**18.11.83 FR 8318433**

(43) Date de publication de la demande:
**22.08.84 Bulletin 84/34**

(45) Mention de la délivrance du brevet:
**27.01.88 Bulletin 88/4**

(84) Etats contractants désignés:
**AT BE CH DE FR GB IT LI LU NL SE**

(56) Documents cités:
**FR - A - 2 141 697**

**CHEMICAL ABSTRACTS, vol. 76, no. 7, 14 février 1972, page 22, no. 41974h, Columbus, Ohio, US M.R. ORNELLAS: "Biochemical studies of cerebral subfractions after chronic administration of 4-methyl-3-(2-(morpholino)ethylamino)-6-phenylpyridazine hydrochloride, AG 620"**
**CHEMICAL ABSTRACTS, vol. 73, no. 1, 6 juillet 1970, page 199, no. 12845z, Columbus, Ohio, US M.R. ORNELLAS et al.: "Pharmacological interpretation of the energy metabolism of rat brain in vivo and in vitro in connection with a study on some pyridazines"**

(73) Titulaire: **SANOFI, société anonyme, 40, Avenue George V, F-75008 Paris (FR)**

(72) Inventeur: **Bizière, Kathleen, 6, Lotissements Rayons d'Oc, F-34170 Clapiers (FR)**
Inventeur: **Kan, Jean-Paul, 25, Lotissement l'Olivette, F-34100 Clapiers (FR)**
Inventeur: **Wermuth, Camille-Georges, 3, rue de la Côte d'Azur, F-67100 Strasbourg (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 116 494 B1

ACTORUM AG

**Description**

Depuis de nombreuses années, des dérivés de la pyridazine ont été proposés en tant que médicaments. Dans un grand nombre de cas, il s'agit de substances actives sur le système cardiovasculaire et présentant en particulier un effet hypotenseur ou vasodilatateur. Plus rarement, on a mentionné, parmi les dérivés de la pyridazine, une action anti-inflammatoire et analgésique. Enfin, le brevet française n° 2 141 697 décrit une famille de produits répondant à la formule générale:

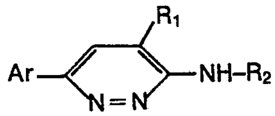

où:

– $R_1$ représente l'hydrogène ou un groupe alkyle inférieur;

– Ar représente un reste aromatique;

– $R_2$ désigne un groupe:

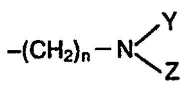

dans lequel n = 2 ou 3 et Y et Z représentent un groupe alkyle inférieur, ou bien

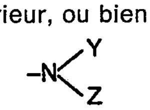

constitue un radical hétérocyclique.

Ces composés sont caractérisés par une activité psychotrope de type psychostimulante.

On a maintenant trouvé que l'introduction du groupement cyano en position 4 de la pyridazine améliore sensiblement les propriétés thérapeutiques de ces produits par rapport à celles décrites pour la même famille de pyridazines non substituées en 4 ou substituées dans la même position par un groupement méthyle dont l'exemple le plus connu est la minaprine (DCI) (Ar = $C_6H_5$, $R_1$ = $CH_3$, $R_2$ = β-morpholinoéthyle).

Ainsi, la présente invention a pour objet, selon un des ses aspects, des cyano-4 pyridazines ayant la formule suivante:

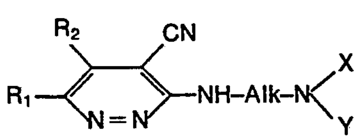

(I)

dans laquelle:

– l'un des substituants $R_1$ ou $R_2$ représente l'hydrogène et l'autre représente l'hydrogène; un groupe alkyle en $C_1$–$C_6$; un groupe cycloalkyle en $C_3$–$C_7$; un groupe phényle; un groupe phényle monosubstitué par un atome d'halogène, un groupe trifluorométhyle, un groupe hydroxy, un groupe alcoxy en $C_1$–$C_6$, un groupe alkyle en $C_1$–$C_6$, un groupe alcanoyloxy en $C_1$–$C_6$, un groupe cyano, un groupe alkylthio en $C_1$–$C_6$, un groupe alkylsulfinyle en $C_1$–$C_6$, un groupe alkylsulfonyle en $C_1$–$C_6$ ou un groupe sulfamyle; un groupe phényle disubstitué par l'un des substituants mentionnés ci-dessus pour le groupe phényle monosubstitué et l'autre substituant étant un atome de chlore ou de fluor ou un groupe méthoxy; un groupe naphtyle-1; un groupe naphtyle-2; un groupe thiényle-2; un groupe thiényle-3; un groupe indolyle-3;

– Alk représente un groupe éthylène, un groupe propylène-1,2 ou un groupe propylène-1,3;

– X désigne l'hydrogène;

– Y représente l'hydrogène ou un groupe β-hydroxyéthyle ou encore le groupe

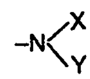

représente un groupe morpholino-4 ou oxo-3 morpholinyle-4, ainsi que leurs sels pharmaceutiquement acceptables.

La présente invention a également pour objet la (morpholino-2 éthylamino)-3 cyano-4 diphényl-5,6 pyridazine, la (morpholino-2 éthylamino)-3 cyano-4 (nitro-4 phényl)-6 pyridazine et leurs sels pharmaceutiquement acceptables.

La comparaison des composés de l'invention avec la minaprine sur plusieurs tests pharmacologiques, mettant en évidence leur activité psychotrope, a montré que les composés selon l'invention ont une dose efficace médiane comparable ou inférieure à celle de la minaprine, alors que leur toxicité est notablement inférieure. Ainsi, les composés selon l'invention présentent un coefficient thérapeutique très supérieur à celui de la minaprine.

Ainsi, la présente invention a pour objet un procédé pour la préparation des composés de formule I dans laquelle $R_1$, $R_2$, Alk, X et Y sont tels que définis ci-dessus, qui est représenté par le schéma suivant:

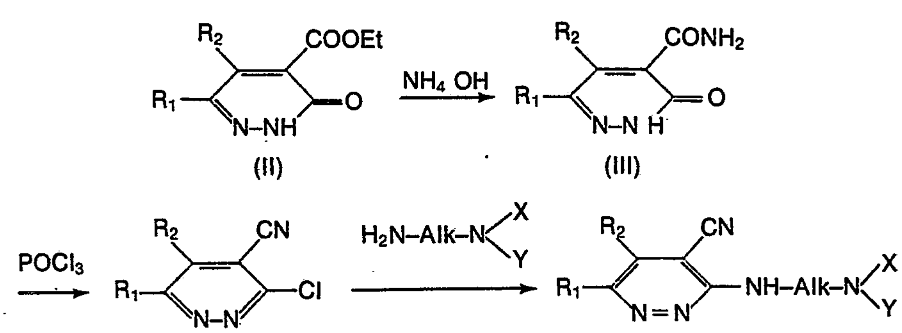


(II) (III) (IV) (I)

Le procédé de la présente invention est caractérisé en ce que l'on effectue les réactions suivantes:

a) on traite une éthoxycarbonyl-4 pyridazone convenablement substituée (II) par l'ammoniaque de façon à obtenir la pyridazone carboxamide-4 correspondante (III);

b) on traite la pyridazone carboxamide-4 par l'oxychlorure de phosphore de façon à obtenir la chloro-3 cyano-4 pyridazine (IV);

c) par action de l'amine de formule

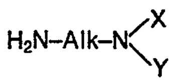

dans laquelle le groupe

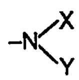

représente soit $-NH_2$, soit le groupe morpholino-4, soit le groupe $-NHCH_2CH_2OH$, on substitue la pyridazine en position 3 pour aboutir au composé de formule (I) correspondant.

Pour obtenir le composé de formule (I) dans laquelle le groupe

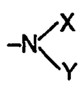

représente le radical oxo-3 morpholinyl, on traite le composé obtenu de formule (I) dans laquelle le groupe

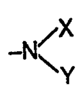

représente le groupe $-NHCH_2CH_2OH$ par du chlorure de chloroacétyle en solution basique et on cyclise le composé obtenu par traitement avec du méthylate de sodium.

Dans la première étape, on utilise une solution aqueuse concentrée d'ammoniaque en excès et on effectue la réaction à température ambiante pendant 10 à 15 h.

La seconde étape est effectuée avec un excès d'oxychlorure de phosphore à une température d'environ 80 °C pendant plusieurs heures.

Enfin, la dernière étape s'effectue par chauffage des deux réactifs au sein d'un solvant convenable, tel que le n-butanol. Les (morpholino-2 éthylamino)-3 cyano-4 diphényl-5,6 pyridazine et (morpholino-2 éthylamino)-3 cyano-4 (nitro-4 phényl)-6 pyridazine sont obtenues selon le même schéma réactionnel.

Les produits de départ sont généralement connus. Ceux qui ne le sont pas peuvent être aisément préparés, par exemple à partir d'une α-halocétone de formule:

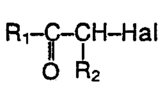

(A)

où Hal est un atome de chlore ou de brome avec du malonate d'éthyle, pour former le malonate substitué:

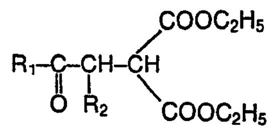

(B)

Celui-ci traité par l'hydrazine conduit au produit de formule:

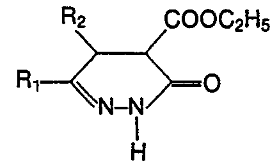

(C)

qui, par déshydrogénation, par exemple à l'aide du brome dans l'acide acétique, fournit le produit II désiré.

Les intermédiaires clés du procédé de la présente invention, ayant les formules III et IV ci-dessus, sont des produits nouveaux lorsqu'au moins l'un des substituants $R_1$ et $R_2$ est autre que l'hydrogène ou le groupe méthyle.

Aussi, la présente invention a pour objet, selon un autre de ses aspects, ces composés de formules III et IV, en tant que produits nouveaux utiles comme intermédiaires.

Enfin, selon encore un autre de ses aspects, la présente invention concerne des compositions pharmaceutiques contenant, en tant que produits actifs, les composés de formule I ci-dessus ou leurs sels pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés, sous formes unitaires d'administration, en mélange avec des supports pharmaceutiques classiques, aux mammifères, y compris l'être humain, pour le traitement de diverses affections neurologiques et psychiatriques: troubles de l'humeur et du comportement, dépressions neurologiques et endogènes, troubles de la mémoire, hyperkinésie infantile, autisme, insuffisances sexuelles d'origine psychogène.

Parmi les formes unitaires d'administration appropriées, il faut citer les formes par voie orale, telles que les comprimés, les gélules, les poudres, les granulés et les solutions ou suspensions orales et les formes d'administration sublinguale et buccale, de même que les formes d'administration parentérales utiles pour une administration sous-cutanée, intramusculaire ou intraveineuse.

Afin d'obtenir l'effet thérapeutique désiré, la dose de principe actif peut varier entre 0,1 et 50 mg par kg de poids corporel et par jour.

Chaque dose unitaire peut contenir de 1 à 500 mg d'ingrédient actif en combinaison avec un support pharmaceutique approprié. Elle peut être administrée de 1 à 4 fois par jour.

Les exemples suivants illustrent l'invention sans toutefois la limiter.

PREPARATION 1:

a) – Phénacyl-malonate d'éthyle

On chauffe au reflux pendant une nuit 240,25 g de malonate d'éthyle, 138 g de carbonate de potassium, 5 g d'iodure de potassium et 154 g de chlorure de phénacyle dans 2 l d'acétone anhydre.

Après avoir filtré les sels minéraux, on évapore à sec le filtrat puis on distille sous pression réduite le malonate d'éthyle en excès (pression: 0,5 mbar; température: environ 60 °C). Le résidu de distillation est chromatographié sur colonne de silice en utilisant comme éluant un mélange cyclohexane-acétate d'éthyle (9/1). Le cétoester attendu se présente sous forme d'une huile rouge. Rendement: 80,3%.

b) – Ethoxycarbonyl-4 phényl-6 4,5-dihydro 2H-pyridazinone-3

On dissout 40,5 g du produit précédemment obtenu dans 70 ml d'éthanol absolu et on ajoute au milieu réactionnel, à une température voisine de 0 °C, goutte à goutte et sous agitation, 7,25 g d'hydrate d'hydrazine. Lorsque le milieu réactionnel est revenu à température ambiante, on agite pendant 24 h puis on filtre le précipité beige obtenu correspondant à la pyridazinone attendue.

Le filtrat est traité par 3,62 g d'hydrate d'hydrazine. Après 24 h d'agitation, une quantité supplémentaire de pyridazinone peut être filtrée. La même opération est encore répétée une fois sur le filtrat.

Après purification par passage sur colonne de silice en utilisant comme éluant le mélange cyclohexane-acétate d'éthyle (volume/volume: 1/1), on obtient le composé attendu avec un rendement de 37%.

c) – Ethoxycarbonyl-4 phényl-6 2H-pyridazinone-3

$$(II)\ R_1 = C_6H_5;\ R_2 = H$$

On dissout 9 g du composé obtenu en b) dans 200 ml d'acide acétique puis on ajoute 11,18 g de brome à la solution, sous agitation. Une décoloration du milieu se produit au bout de 5 min. Après 2 h à température ambiante et sous agitation, on verse dans 200 ml d'eau puis on extrait au chlorure de méthylène et on évapore à sec la phase organique.

Le résidu est repris trois fois avec du cyclohexane. La poudre beige obtenue est chromatographiée sur colonne de silice en utilisant comme éluant un mélange cyclohexane-acétate d'éthyle (volume/volume: 1/1). La pyridazinone attendue est obtenue avec un rendement de 51%. F. 150 °C.

PREPARATIONS 2 à 8

En opérant comme décrit dans la préparation 1, en partant de:

parachlorophénylchlorométhylcétone,
parafluorophénylchlorométhylcétone,
α-naphtylbromométhylcétone,
cyclohexylchlorométhylcétone,
dichloro-2,4 phénylchlorométhylcétone,
indolyl-3 chlorométhylcétone,
thiényl-3 chlorométhylcétone,

par réaction avec du malonate d'éthyle, condensation avec de l'hydrazine hydratée et déshydrogénation avec du brome dans l'acide acétique, on obtient les produits (II) décrits dans le tableau 1.

Tableau 1

| Préparation n° | Composés II R1 | R2 |
|---|---|---|
| 2 | 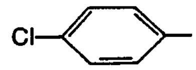 | H |
| 3 | 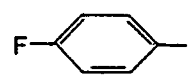 | H |
| 4 | 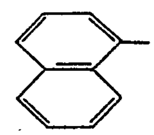 | H |
| 5 | 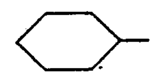 | H |
| 6 | 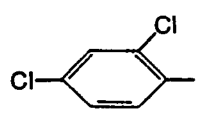 | H |
| 7 | 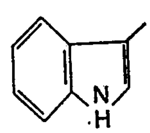 | H |
| 8 | 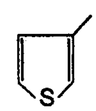 | H |

PREPARATIONS 9 à 18

En opérant comme décrit dans la préparation 1 en partant de:

méthoxy-4 phénylchlorométhylcétone,
hydroxa-4 phénylchlorométhylcétone,
diméthoxy-3,4 phénylchlorométhylcétone,
nitro-4 phénylchlorométhylcétone,
méthyl-3 phénylchlorométhylcétone,
cyclopentylchlorométhylcétone,
trifluorométhyl-3 phénylchlorométhylcétone,
phénylchloro-1 éthylcétone,
méthyl-α-chlorobenzylcétone,
α-chlorophénylacétaldéhyde

par action avec du malonate d'éthyle, condensation avec de l'hydrazine hydratée et déshydrogénation avec du brome dans l'acide acétique, on obtient les produits décrits dans le tableau 2.

Tableau 2

| Préparation n° | Composés II R1 | R2 |
|---|---|---|
| 9 | 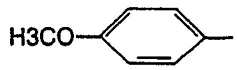 | H |
| 10 | 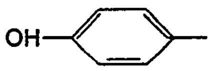 | H |
| 11 | 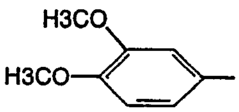 | H |
| 12 | 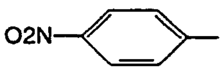 | H |
| 13 | 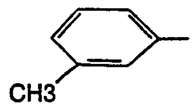 | H |
| 14 | 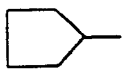 | H |
| 15 | 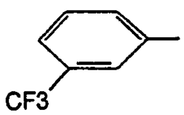 | H |
| 16 | 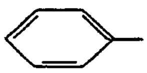 | CH3 |
| 17 | CH3 | 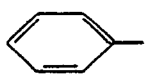 |
| 18 | H | 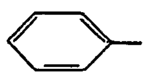 |

PREPARATIONS 19 à 36

De la même façon, à partir de:
méthylthio-4 phénylchlorométhylcétone,
méthylsulfinyl-4 phénylchlorométhylcétone,
méthylsulfonyl-4 phénylchlorométhylcétone,
naphtyl-2 chlorométhylcétone,
thiényl-2 chlorométhylcétone,
chloro-2 phénylchlorométhylcétone,
chloro-3 phénylchlorométhylcétone,
dichloro-3,4 phénylchlorométhylcétone,
cyclopropylchlorométhylcétone,
méthyl-4 phénylchlorométhylcétone,
méthyl-2 phénylchlorométhylcétone,
trifluorométhyl-4 phénylchlorométhylcétone,
cyclooctylchlorométhylcétone,
cyano-4 phénylchlorométhylcétone,
sulfamoyl-4 phénylchlorométhylcétone,
dihydroxy-3,4 phénylchlorométhylcétone,
acétoxy-4 phénylchlorométhylcétone,
α-bromodésoxybenzoïne,
on obtient les produits mentionnés dans le tableau 3.

Tableau 3

| Préparation n° | Composés II R1 | R2 |
|---|---|---|
| 19 | 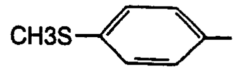 | H |
| 20 | 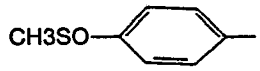 | H |
| 21 | 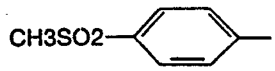 | H |
| 22 | 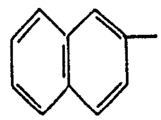 | H |
| 23 | 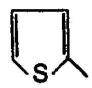 | H |
| 24 | 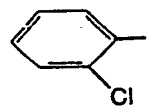 | H |
| 25 | 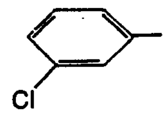 | H |
| 26 | 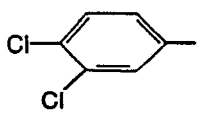 | H |
| 27 | 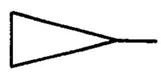 | H |
| 28 | 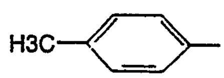 | H |
| 29 | 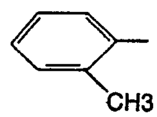 | H |
| 30 | 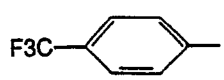 | H |
| 32 | 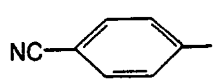 | H |
| 33 | 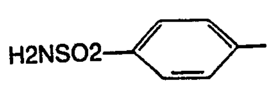 | H |
| 35 | 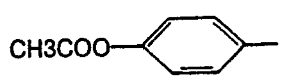 | H |
| 36 | 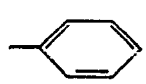 | 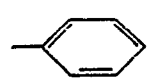 |

EXEMPLE 1
Phényl-6 oxo-3 2H-pyridazine carboxamide-4

(III) $R_1 = C_6H_5$; $R_2 = H$

A 40 ml d'une solution d'ammoniaque concentrée, on ajoute 2 g du produit obtenu dans la préparation 1 et on laisse sous agitation pendant une nuit à température ambiante. On essore le solide et on sèche pour obtenir le produit attendu.
Rendement 86%; F. > 300 °C.

EXEMPLES 2 à 17
En opérant comme dans l'exemple 1 à partir des dérivés éthoxycarbonyle correspondants, on obtient les composés III décrits dans le tableau 4.

Tableau 4

| Exemple n° | Composés III R1 | R2 |
|---|---|---|
| 2 | 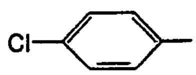 | H |
| 3 | 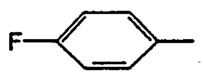 | H |
| 4 | 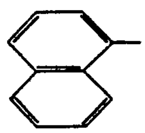 | H |
| 5 | 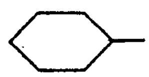 | H |
| 6 | 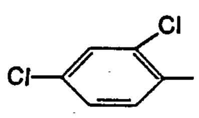 | H |
| 7 | 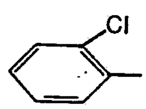 | H |
| 8 | 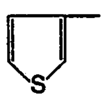 | H |
| 9 | 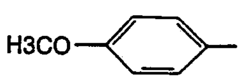 | H |
| 10 | 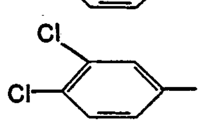 | H |
| 11 | 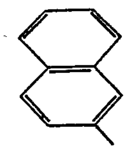 | H |
| 12 | 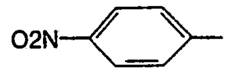 | H |
| 13 | 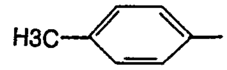 | H |
| 14 | 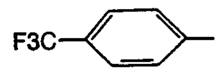 | H |
| 15 | 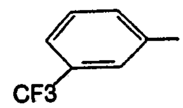 | H |
| 16 | 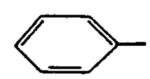 | 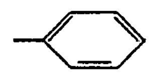 |
| 17 | 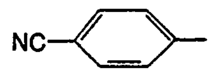 | H |

EXEMPLE 18
Chloro-3 cyano-4 phényl-6 pyridazine

(IV) $R_1 = C_6H_5$; $R_2 = H$)

On dissout 1,5 g du produit obtenu à l'exemple 1 dans 20 ml d'oxychlorure de phosphore puis on chauffe à 80 °C pendant 5 h. On verse le mélange dans 50 ml d'eau. Il apparaît un précipité qu'on essore et sèche.
Rendement: 58,3%; F. 206 °C.

EXEMPLES 19 à 34
En opérant comme dans l'exemple 18 à partir des amides correspondants de formule III, on obtient les chloro-3 cyano-4 pyridazines de formule IV décrites dans le tableau 5.

Tableau 5

| Exemple n° | Composés IV R1 | R2 | Pt de fusion ou Rf |
|---|---|---|---|
| 19 | 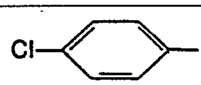 | H | Chromatographie |
| 20 | 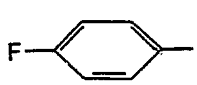 | H | F : 170 °C |

Tableau 5 (suite)

| Exemple n° | Composés IV R1 | R2 | Pt de fusion ou Rf |
|---|---|---|---|
| 21 | 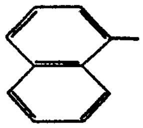 | H | Chromatographie |
| 22 | 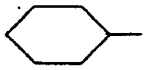 | H | Rf : 0,9 (hexane-acétate d'éthyle) |
| 23 | 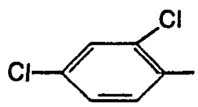 | H | F : 152–154 °C |
| 24 | 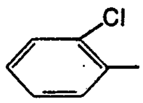 | H | Rf : 0,4 (hexane–acétate d'éthyle 211) |
| 25 | 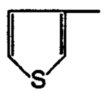 | H | F : 152 °C |
| 26 | 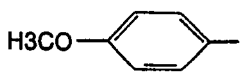 | H | F : 196–198 °C |
| 27 | 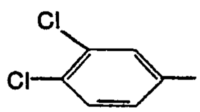 | H | F : 204–206 °C |
| 28 | 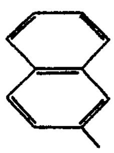 | H | F : 231–232 °C |
| 29 | 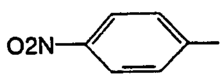 | H | Rf : 0,8 (hexane–acétate d'éthyle 211) |
| 30 | 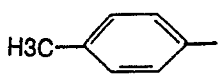 | H | F : 191 °C |
| 31 | 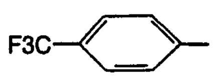 | H | Rf : 0,7 (hexane–acétate d'éthyle 1/1) |
| 32 | 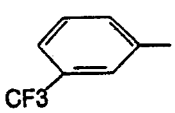 | H | Rf : 0,8 (hexane–acétate d'éthyle 1/1) |
| 33 | 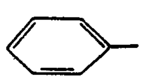 | 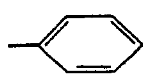 | – |
| 34 | 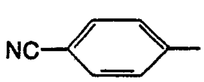 | H | Rf : 0,9 (hexane–acétate d'éthyle 1/1, vol/vol) |

EXEMPLE 35

(Morpholino-2 éthylamino)-3 cyano-4 phényl-6 pyridazine, dichlorhydrate SR 95 191

(I) $R_1 = C_6H_5$; $R_2 = H$; $Alk = (CH_2)_2$;

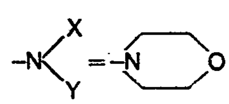

On dissout 7,3 g du composé chloré de l'exemple 18 dans 60 ml de butanol normal et on ajoute

8 g de N-(amino-2 éthyl)-morpholine. On chauffe au reflux pendant 3 h puis on verse le mélange dans 1000 ml d'eau. On extrait la phase organique avec de l'éther puis on extrait la solution éthérée avec une solution d'acide sulfurique 1N. On sépare la phase aqueuse, on alcalinise par de la soude et on extrait avec de l'éther. On sèche la phase éthérée sur sulfate de magnésium puis on évapore le solvant à siccité sous vide. On obtient un solide jaune. Rendement 81,3%; F. 138 °C.

On dissout 6,8 g du produit obtenu ci-dessus dans 100 ml de méthanol sec et on fait barboter un courant de gaz chlorhydrique. On évapore le solvant à siccité sous vide et on reprend le résidu par de l'éther anhydre.

Il se forme un précipité de dichlorhydrate de (morpholino-2 éthylamino)-3 cyano-4 phényl-6 pyridazine qui est recristallisé 2 fois dans de l'isopropanol. F. 144 °C (décomposition).

A partir de la base; on peut de même préparer les sels suivants du même composé:

| | |
|---|---|
| Monocitrate | F. 181 °C (éthanol aqueux) |
| Diglutamate | F. supérieur à 260 °C (éthanol aqueux) |
| Monochlorhydrate | F. 230 °C |
| Monofumarate | F. 204 °C (acétone) |
| Monomaléate | F. 168 °C (acétone) |

EXEMPLES 36 à 51

En opérant comme décrit ci-dessus, par réaction du dérivé chloré correspondant de formule (IV) avec la N-(amino-2 éthyl)-morpholine, on obtient les composés (I) décrits dans le tableau 6.

Tableau 6

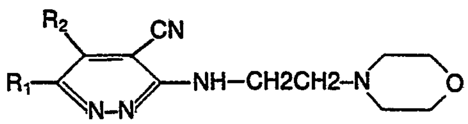

| Exemple n° | N° Code Produit SR | R1 | R2 | Sel ou Base Point de fusion (°C) (solvant) |
|---|---|---|---|---|
| 36 | 95276 A | 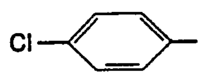 | H | Dichlorhydrate 135–140 (décomposition) |
| 37 | 95306 | 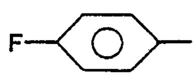 | H | Base 208 |
| 38 | 95294 A | 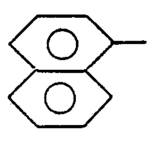 | H | Dichlorhydrate 130–140 (décomposition isopropanol) |
| 39 | 95331 A | 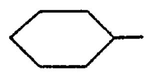 | H | Dichlorhydrate 168 (décomposition) |
| 40 | 42632 A | 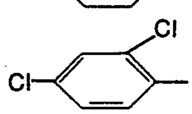 | H | Fumarate (2/3) 183–185 (acétone) avec 0,5 H2O |
| 41 | 95324 A | 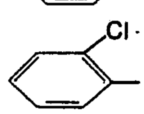 | H | Dichlorhydrate 228 (isopropanol) |
| 42 | 95274 A | 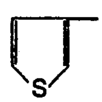 | H | Dichlorhydrate 170 (méthanol) |
| 43 | 42595 A | 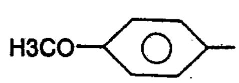 | H | Fumarate (1/1) 240–242 (acétone) |
| 44 | 42638 A | 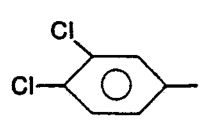 | H | Maléate (1/1) 240–242 (acétone) |

Tableau 6 (suite)

| Exemple n° | N° Code Produit SR | R1 | R2 | Sel ou Base Point de fusion (°C) (solvant) |
|---|---|---|---|---|
| 45 | 42692 | 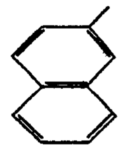 | H | Base 184–186 (isopropanol) |
| 46 | 95323 A | 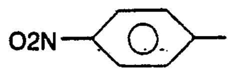 | H | Dichlorhydrate 260 (décomposition) |
| 47 | 42639 | 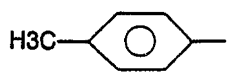 | H | Base 150–151 (isopropanol) |
| 48 | 95330 A | 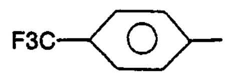 | H | Dichlorhydrate 278 Base 159 |
| 49 | 95328 | 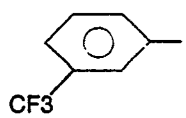 | H | Base 129 |
| 50 | 95071 A | 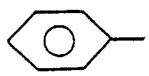 | 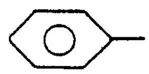 | Monochlorhydrate 210 (décomposition) |
| 51 | 95329 | 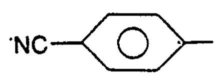 | H | Base 205 (méthanol) |

EXEMPLES 52 à 56

En opérant comme dans l'exemple 35 mais en faisant varier le composé aminé utilisé, on obtient à partir de différentes chloro-3 pyridazines les composés (I) réunis dans le tableau 7.

Tableau 7

| Exemple n° | Numéro de code SR | R1 | R2 | Alk | 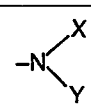 | Base ou sel point de fusion, °C |
|---|---|---|---|---|---|---|
| 54 | 95332 A | 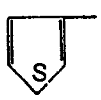 | H | ″ | ″ | Dichlorhydrate 124 (décomposition) |
| 56 | 42633 | 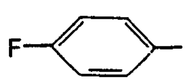 | H | (CH2)2 | –NH2 | Base 200–202 (éthanol) |

EXEMPLE 57

[(Oxo-3 morpholino)-2 éthylamino]-3 cyano-4 phényl-6 pyridazine, chlorhydrate; SR 95 327A

$R_1 = C_6H_5$; $R_2 = H$; $Alk = (CH_2)_2$;

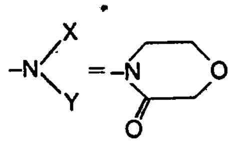

a) A la solution de 3 g de ((β-hydroxyéthyl-amino)-2 éthyl amino)-3 cyano-4 phényl-6 pyridazine (obtenue en opérant comme dans l'exemple 35 mais en faisant varier le composé aminé utilisé (ici: $H_2N-(CH_2)_2-NHCH_2CH_2OH$)) dans 54 ml de dichlorométhane, on ajoute la solution de 4,2 g de soude dans 54 ml d'eau puis, sous agitation on refroidit à −5 °C, −10 °C. On ajoute lentement 1,17 g de chlorure de chloroacétyle puis on laisse la température remonter à 20 °C et on laisse 15 h sous agitation à cette température. On sépare la

phase organique et on évapore à siccité sous vide.

On obtient un solide jaune utilisé tel quel pour l'étape suivante:

b) On dissout le produit obtenu ci-dessus dans 27 ml de méthanol anhydre, et on ajoute la solution de méthylate de sodium obtenue par action de 0,24 g de sodium sur 27 ml de méthanol anhydre. On chauffe au reflux pendant 6 h et on évapore à siccité. On reprend le résidu dans de l'eau et on l'extrait avec de l'acétate d'éthyle. On sépare la couche organique, on sèche sur sulfate de sodium et on évapore à siccité. On purifie le produit par chromatographie sur colonne de silice en éluant avec le mélange acétate d'éthyle-méthanol 8/2, vol/vol.

On obtient une huile jaune pâle (1,5 g). On la dissout dans le méthanol et on fait barboter de l'acide chlorhydrique gazeux sec. On évapore à siccité et on reprend le résidu dans le minimum de méthanol. On ajoute de l'éther anhydre et on essore le précipité de chlorhydrate; F. 128 °C.

EXEMPLE 58

Préparation galénique

A titre d'exemple de préparation galénique, on peut indiquer des gélules contenant:

| | |
|---|---|
| Principe actif | 50 mg |
| Aerosil | 0,5 mg |
| Stéarate de magnésium | 1,5 mg |
| Amidon STA RX 1500 | 48 mg |
| | 100 mg |

L'activité psychotrope d'un composé représentatif de l'invention, à savoir le composé SR 95191 (exemple 37), a été mesurée sur trois tests de pharmacologie en comparaison avec la minaprine et imipramine, antidépresseur très utilisé. De même, la toxicité du produit a été comparée avec celle des produits de référence.

COMPORTEMENT DE DESESPOIR

Ce test a été réalisé chez la souris femelle CDI (Charles River) pesant 18 à 23 g selon la méthode décrite par PORSOLT (Archives Internationales de Pharmacodynamie, 1977, 327–336).

Le principe de ce test est le suivant:
lorsqu'une souris est placée dans un récipient étroit, rempli d'eau, elle se débat puis, au bout de 2 à 4 min, elle s'immobilise et flotte sur le ventre, le dos arrondi, les pattes postérieures ramenées sous le corps et elle ne fait que quelques mouvements nécessaires pour se maintenir la tête hors de l'eau. C'est la réaction dite de désespoir (despair reaction).

Certains psychotropes, notamment les antidépresseurs, allongent le temps pendant lequel la souris se débat.

Le protocole suivant a été choisi:

Les produits à étudier ont été administrés i.p. 1 h avant le test. Pour le test, les animaux sont placés dans un récipient étroit (10 × 10 × 10 cm), rempli d'eau, sur une hauteur de 6 cm, la température de 24 °C plus ou moins 2 °C. Les animaux sont laissés 6 min dans l'eau et on mesure le temps où l'animal reste immobile entre la 2$^{e}$ et la 6$^{e}$ min. Plus ce temps est court, plus la substance est active.

Chaque substance a été étudiée sur un lot de 10 souris. Les résultats sont la moyenne d'au moins deux expériences.

ANTAGONISME DE LA PTOSE INDUITE PAR LA RESERPINE:

Ce test, décrit par GOURET (Journal de pharmacologie Paris 1973, 4 (1), 105–128), a été réalisé chez la souris femelle CDI (Charles River) pesant 20 g plus ou moins 1. La réserpine entraîne un ptôsis 1 heure après son administration intraveineuse; certains antidépresseurs s'opposent à ce ptôsis.

Le protocole suivant a été choisi:

Les substances à étudier ont été administrées i.p. La réserpine est administrée simultanément par voie intraveineuse à la dose de 2 mg/kg. 1 heure après l'administration de réserpine, on note le nombre d'animaux ne présentant pas de ptôsis.

Ce test a été réalisé sur des lots de 10 souris, les résultats sont exprimés en pourcentage d'animaux ne présentant pas de ptôsis et sont la moyenne de, au moins, deux expériences.

COMPORTEMENT ROTATOIRE:

Ce test est décrit par PROTAIS et coll. dans le Journal de pharmacologie, 1976, 7, 251–255.

Des souris femelles Charles River CDI pesant de 20 à 24 g font préalablement l'objet d'une lésion unilatérale du striatum par injection stéréotaxique de 6-hydroxydopamine à raison de 8 µg par animal. Une semaine après cette opération, le produit est administré par voie i.p. à des groupes de 7 souris. Le nombre de rotations est évalué pendant 2 minutes, 1 heure après l'administration du produit. Les rotations ipsilatérales de la lésion sont comptées positivement, celles contralatérales sont comptées négativement. La somme algébrique des rotations pour un groupe d'animaux traités est comparée à celle du groupe d'animaux témoins n'ayant reçu que le véhicule (sérum physiologique).

TOXICITE AIGUE:

Les produits à étudier ont été administrés par voie intrapéritonéale à doses croissantes à des lots de 10 souris. La mortalité provoquée par les produits étudiés a été notée pendant les 24 h ayant suivi l'administration du produit.

A partir des résultats obtenus, on détermine pour chacun des produits étudiés la dose létale 50, c'est-à-dire la dose provoquant la mort de 50% des animaux étudiés.

Les résultats obtenus figurent dans le tableau 8.

Tableau 8

| Composé | Toxicité, voie i.p. DL 50 | Ptôse à la réserpine voie i.p. $DE_{50}$ | Comportement de désespoir voie i.p. | Comportement rotatoire voie i.p. |
|---|---|---|---|---|
| SR 95191 | 250 mg/kg | 3,9 mg/kg | 5 mg/kg : –26% + + | 0,1 mg/kg : –60% + +<br>2 mg/kg : –107% + + |
| Minaprine | 63 mg/kg | 5 mg/kg | 5 mg/kg : –31% + + | 0,125 mg/kg : –53% + +<br>2 mg/kg : –82% + + |
| Iminaprine | 89 mg/kg | 2,4 mg/kg | 10 mg/kg : –38% + + | 3 mg/kg : 6% n.s. |

+ +: $p < 0,1$ test de Student
n.s. : non significatif

De la même manière on a déterminé l'activité psychotrope de deux autres composés représentatifs de l'invention, à savoir les composés SR 95274 A (exemple 42) et SR 95294 A (exemple 38) sur deux des tests de pharmacologie ci-dessus: comportement rotatoire et antagonisme de la ptose induite par la réserpine. Les résultats obtenus sont indiqués dans le tableau 9 ci-après ainsi que la toxicité de ces produits administrés par voie intrapéritonéale dans les conditions indiquées ci-dessus.

Tableau 9

| Produits | Toxicité voie i.p. | Test de l'antogonisme de la ptôse à la réserpine voie i.p. | Comportement rotatoire chez la souris (voie i.p.) |
|---|---|---|---|
| SR 95 274 A | $DL_{50}$ > 300 mg/kg | $DE_{50}$ = 2,6 mg/kg | 0,5 mg/kg –60% *<br>2 mg/kg –82% * |
| SR 95 294 A | | $DE_{50}$ = 10 mg/kg | 0,5 mg/kg –74% *<br>2 mg/kg –92% * |
| Minaprine | $DL_{50}$ = 63 mg/kg | $DE_{50}$ = 5 mg/kg | 2 mg/kg –82% * |

* $p < 0,01$, test de Student

**Revendications**
pour les Etats contractants BE CH DE FR GB IT LI LU NL SE

1. A titre de composés nouveaux, les cyano-4 pyridazines répondant à la formule générale:

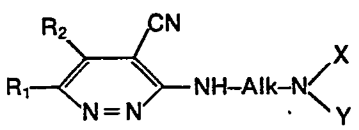
(I)

dans laquelle:

– l'un des substituants $R_1$ ou $R_2$ représente l'hydrogène et l'autre représente l'hydrogène; un groupe alkyle en $C_1$–$C_6$; un groupe cycloalkyle en $C_3$–$C_7$; un groupe phényle; un groupe phényle monosubstitué par un atome d'halogène, un groupe trifluorométhyle, un groupe hydroxy, un groupe alcoxy en $C_1$–$C_6$, un groupe alkyle en $C_1$–$C_6$, un groupe alcanoyloxy en $C_1$–$C_6$, un groupe cyano, un groupe alkylthio en $C_1$–$C_6$, un groupe alkylsulfinyle en $C_1$–$C_6$, un groupe alkylsulfonyle en $C_1$–$C_6$ ou un groupe sulfamyle; un groupe phényle disubstitué par l'un des substituants mentionnés ci-dessus pour le groupe phényle monosubstitué et l'autre étant un atome de chlore ou de fluor ou un groupe méthoxy; un groupe naphtyle-1; un groupe naphtyle-2; un groupe thiényle-2; un groupe thiényle-3; un groupe indolyle-3;

– Alk représente un groupe éthylène, un groupe propylène-1,2 ou un groupe propylène-1,3;

– X désigne l'hydrogène;

– Y représente l'hydrogène ou un groupe β-hydroxyéthyle

le groupe

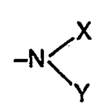

représente un groupe morpholino-4 ou oxo-3 morpholinyle-4, ainsi que de leurs sels pharmaceutiquement acceptables.

2. (Morpholino-2 éthylamino)-3 cyano-4 diphényl-5,6 pyridazine ou un de ses sels pharmaceutiquement acceptables.

3. (Morpholino-2 éthylamino)-3 cyano-4 (nitro-4 phényl)-6 pyridazine ou un de ses sels pharmaceutiquement acceptables.

4. Procédé pour l'obtention des cyano-4 pyridazines selon la revendication 1, caractérisé en ce qu'il consiste:

a) à traiter une éthoxycarbonyl-4 pyridazone convenablement substituée répondant à la formule

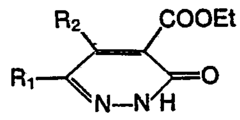

(II)

par de l'ammoniaque de façon à obtenir la carboxamide-4 pyridazone de formule (III)

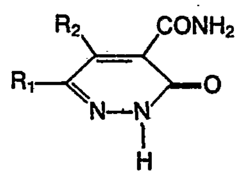

(III)

b) à traiter la carboxamide-4 pyridazone par de l'oxychlorure de phosphore pour obtenir la chloro-3 cyano-4 pyridazine de formule IV

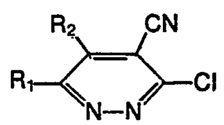

(IV)

c) à faire réagir ladite chloro-3 cyano-4 pyridazine avec l'amine de formule

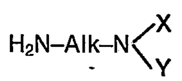

,

dans laquelle Alk est tel que défini à la revendication 1 et le groupe

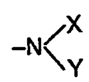

représente soit $-NH_2$, soit le groupe morpholino-4, soit le groupe $-NHCH_2CH_2OH$, à isoler le composé de formule (I) ainsi obtenu sous forme de base libre ou sous forme de sel; éventuellement à traiter le composé obtenu de formule (I) dans laquelle le groupe

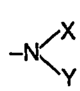

est $-NHCH_2CH_2OH$ par du chlorure de chloroacétyle en solution basique et à cycliser le composé ainsi obtenu par traitement avec du méthylate de sodium pour obtenir le produit de formule (I) dans laquelle le radical

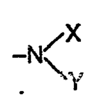

représente un radical oxo-3 morpholinyl; ledit produit étant isolé sous forme de base ou sous forme de sel; les radicaux $R_1$, $R_2$ dans les formules II à IV ci-dessus étant tels que définis dans la revendication 1.

5. Procédé pour l'obtention de la cyano-4 pyridazine selon la revendication 2, caractérisé en ce qu'il consiste:

a) à traiter l'éthoxycarbonyl-4 pyridazone de formule:

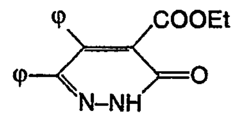

. (II)

par de l'ammoniaque de façon à obtenir la carboxamide-4 pyridazone de formule (III)

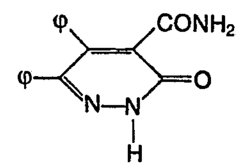

b) à traiter la carboxamide-4 pyridazone par le l'oxychlorure de phosphore pour obtenir la chloro-3 cyano-4 pyridazine de formule (IV)

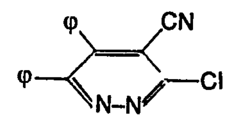

c) à faire réagir celle-ci avec l'amine de formule

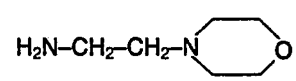

,

pour former le composé attendu, celui-ci étant isolé sous forme de base libre ou sous forme de sel.

6. Procédé pour l'obtention de la cyano-4 pyridazine selon la revendication 3, caractérisé en ce qu'il consiste:

a) à traiter l'éthoxycarbonyl-4 pyridazone de formule

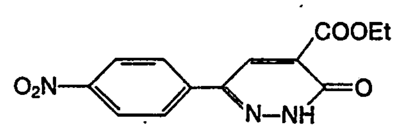

(II)

par de l'ammoniaque de façon à obtenir la carboxamide-4 pyridazone de formule

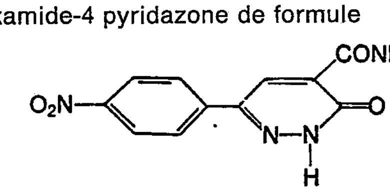

(III)

b) à traiter la carboxamide-4 pyridazone par de l'oxychlorure de phosphore pour obtenir la chloro-3 cyano-4 pyridazine de formule (IV)

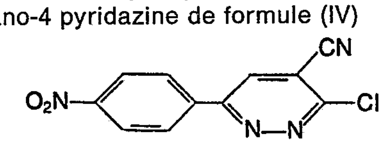

(IV)

c) à faire réagir celle-ci avec l'amine de formule

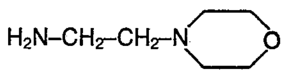 ,

pour former le composé attendu celui-ci étant isolé sous forme de base libre ou sous forme de sel.

7. Procédé selon l'une quelconque des revendications 4 à 6, caractérisé en ce que les éthoxycarbonyl-4 pyridazones de formule (II) sont préparées à partir d'une α-halocétone de formule

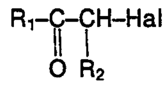

dans laquelle Hal est un atome de chlore ou de brome, $R_1$ et $R_2$ sont tels que définis à la revendication 1 ou $R_1$ et $R_2$ représentent un groupe phényle ou $R_1$ représente un groupe nitro-4-phényle tandis que $R_2$ représente l'hydrogène; avec du malonate d'éthyle pour former le malonate substitué:

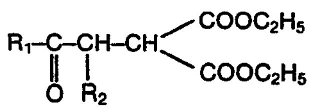

lequel est ensuite cyclisé par action de l'hydrazine pour obtenir le composé de formule

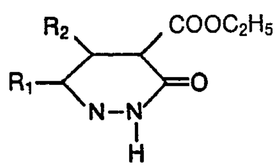 (C)

qui est alors déshydrogéné pour former le composé de formule II désiré.

8. A titre de composés intermédiaires pour la synthèse des cyano-4 pyridazines par l'un quelconque des procédés selon l'une des revendications 4 à 6, les composés de formule IV ci-après

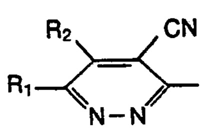 (IV)

dans lesquels $R_1$ et $R_2$ sont tels que définis à la revendication 1, ou $R_1$ et $R_2$ représentent un groupe phényle ou $R_1$ représente un groupe nitro-4 phényle tandis que $R_2$ représente l'hydrogène, à la condition que, au moins, l'un des groupes $R_1$ ou $R_2$ soit autre que l'hydrogène ou le groupe méthyle.

9. Procédé pour l'obtention des cyano-4 pyridazines selon la revendication 8, caractérisé en ce qu'il consiste:

a) à traiter une éthoxycarbonyl-4 pyridazone convenablement substituée, de formule

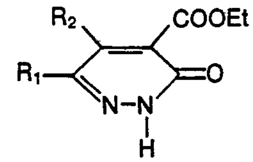

par de l'ammoniaque de façon à obtenir la carboxamide-4 pyridazone de formule (III)

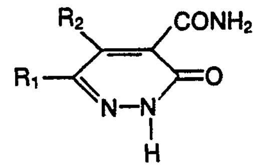

b) à traiter ladite carboxamide-4 pyridazone par de l'oxychlorure de phosphore; les radicaux $R_1$ et $R_2$ étant tels que définis à la revendication 8.

10. Compositions pharmaceutiques, caractérisées en ce qu'elles contiennent à titre d'ingrédient actif au moins une cyano-4 pyridazine selon l'une quelconque des revendications 1 à 3, en combinaison avec un support pharmaceutique approprié.

11. Compositions pharmaceutiques, ayant une action psychotrope, caractérisées en ce qu'elles contiennent à titre d'ingrédient actif au moins une cyano-4 pyridazine selon l'une quelconque des revendications 1 à 3, en combinaison avec un support pharmaceutique approprié.

12. Compositions pharmaceutiques, selon la revendication 11, caractérisées en ce qu'elles sont conditionnées sous forme de doses unitaires, pour l'administration orale, contenant 1 à 500 mg d'ingrédient actif.

**Revendications**
pour l'Etat contractant AT

1. Procédé pour l'obtention des cyano-4 pyridazines répondant à la formule générale:

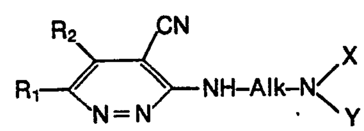 (I)

dans laquelle:

– l'un des substituants $R_1$ ou $R_2$ représente l'hydrogène et l'autre représente l'hydrogène; un groupe alkyle en $C_1$–$C_6$; un groupe cycloalkyle en $C_3$–$C_7$; un groupe phényle; un groupe phényle monosubstitué par un atome d'halogène, un groupe trifluorométhyle, un groupe hydroxy, un groupe alcoxy en $C_1$–$C_6$, un groupe alkyle en $C_1$–$C_6$, un groupe alcanoyloxy en $C_1$–$C_6$, un groupe cyano, un groupe alkylthio en $C_1$–$C_6$, un groupe alkylsulfinyle en $C_1$–$C_6$, un groupe alkylsulfonyle en $C_1$–$C_6$ ou un groupe sulfamyle; un groupe phényle disubstitué par l'un des substituants mentionnés ci-dessus pour le groupe phényle monosubstitué et l'autre substituant étant un atome de chlore ou de fluor ou un groupe méthoxy; un groupe naphtyle-1; un groupe naphtyle-2; un groupe thiényle-2; un groupe thiényle-3; un groupe indolyle-3;

– Alk représente un groupe éthylène, un groupe propylène-1,2 ou un groupe propylène-1,3;

– X désigne l'hydrogène;

– Y représente l'hydrogène ou un groupe β-hydroxyéthyle ou encore le groupe

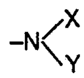

représente un groupe morpholino-4 ou oxo-3 morpholinyle-4, ainsi que de leurs sels pharmaceutiquement acceptables, caractérisé en ce qu'il consiste:

a) à traiter une éthoxycarbonyl-4 pyridazone convenablement substituée répondant à la formule

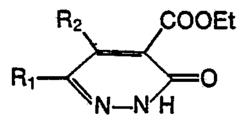

(II)

par de l'ammoniaque de façon à obtenir la carboxamide-4 pyridazone de formule (III)

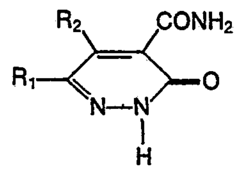

(III)

b) à traiter la carboxamide-4 pyridazone par de l'oxychlorure de phosphore pour obtenir la chloro-3 cyano-4 pyridazine de formule IV

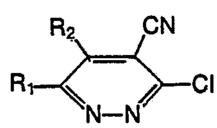

(IV)

c) à faire réagir ladite chloro-3 cyano-4 pyridazine avec l'amine de formule

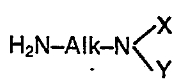 ,

dans laquelle Alk est tel que défini ci-dessus et le groupe

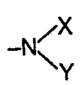

représente soit $-NH_2$, soit le groupe morpholino-4, soit le groupe $-NHCH_2CH_2OH$, à isoler le composé de formule (I) ainsi obtenu sous forme de base libre ou sous forme de sel; éventuellement à traiter le composé obtenu de formule (I) dans laquelle

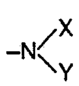

est $-NHCH_2CH_2OH$ par du chlorure de chloroacétyle en solution basique et à cycliser le composé ainsi obtenu par traitement avec du méthylate de sodium pour obtenir le produit de formule (I) dans laquelle le radical

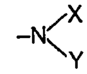

représente un radical oxo-3 morpholinyl; ledit produit étant isolé sous forme de base ou sous forme de sel; les radicaux $R_1$, $R_2$ dans les formules II à IV ci-dessus étant tels que définis ci-dessus.

2. Procédé pour l'obtention de la (morpholino-2 éthylamino)-3 cyano-4 diphényl-5,6 pyridazine ou de l'un de ses sels pharmaceutiquement acceptables, caractérisé en ce qu'il consiste:

a) à traiter l'éthoxycarbonyl-4 pyridazone de formule:

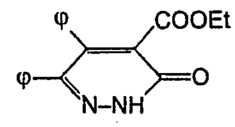

. (II)

par de l'ammoniaque de façon à obtenir la carboxamide-4 pyridazone de formule (III)

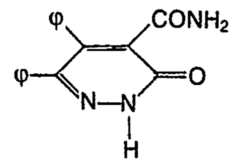

b) à traiter la carboxamide-4 pyridazone par de l'oxychlorure de phosphore pour obtenir la chloro-3 cyano-4 pyridazine de formule (IV)

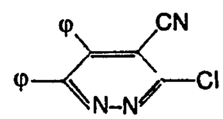

c) à faire réagir celle-ci avec l'amine de formule

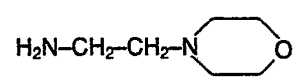

pour former le composé attendu, celui-ci étant isolé sous forme de base libre ou sous forme de sel.

3. Procédé pour l'obtention de la (morpholino-2 éthylamino)-3 cyano-4 (nitro-4 phényl)-6 pyridazine ou de l'un de ses sels pharmaceutiquement acceptables, caractérisé en ce qu'il consiste:

a) à traiter l'éthoxycarbonyl-4 pyridazone de formule:

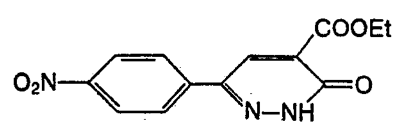

(II)

par de l'ammoniaque de façon à obtenir la carboxamide-4 pyridazone de formule

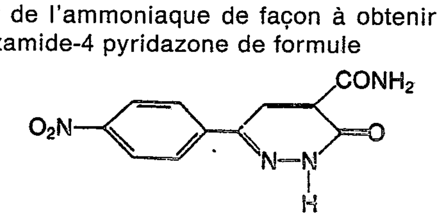

(III)

b) à traiter la carboxamide-4 pyridazone par de l'oxychlorure de phosphore pour obtenir la chloro-3 cyano-4 pyridazine de formule (IV)

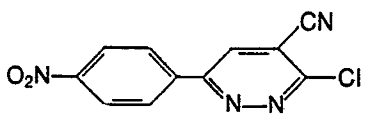

(IV)

c) à faire réagir celle-ci avec l'amine de formule

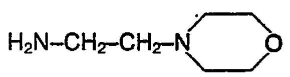

pour former le composé attendu, celui-ci étant isolé sous forme de base libre ou sous forme de sel.

4. Procédé selon l'une quelconque des revendications 1 à 3, caractérisé en ce que les éthoxycarbonyl-4 pyridazones de formule (II) sont préparées à partir d'une α-halocétone de formule

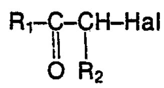

dans laquelle Hal est un atome de chlore ou de brome, $R_1$ et $R_2$ sont tels que définis à la revendication 1 ou $R_1$ et $R_2$ représentent chacun un groupe phényle ou $R_1$ représente un groupe nitro-4-phényle tandis que $R_2$ représente l'hydrogène; avec du malonate d'éthyle pour former le malonate substitué;

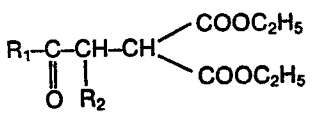

lequel est ensuite cyclisé par action de l'hydrazine pour obtenir le composé de formule

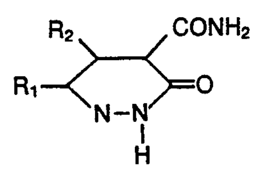

(C)

qui est alors déshydrogéné pour former le composé de formule (II) désiré.

5. Procédé pour l'obtention des composés de formule (IV) ci-après

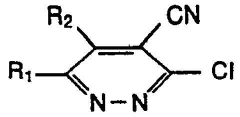

(IV)

dans laquelle $R_1$ et $R_2$ sont tels que définis à la revendication 1, ou $R_1$ et $R_2$ représentent chacun un groupe phényle ou $R_1$ représente un groupe nitro-4 phényle tandis que $R_2$ représente l'hydrogène, à la condition que, au moins, l'un des groupes $R_1$ ou $R_2$ soit autre que l'hydrogène ou le groupe méthyle, caractérisé en ce qu'il consiste:

a) à traiter une éthoxycarbonyl-4 pyridazone convenablement substituée de formule

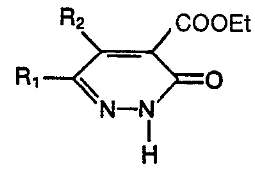

par de l'ammoniaque de façon à obtenir la carboxamide-4 pyridazone de formule (III)

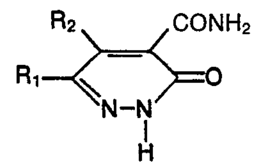

b) à traiter ladite carboxamide-4 pyridazone par de l'oxychlorure de phosphore; les radicaux $R_1$ et $R_2$ étant tels que définis ci-dessus.

6. Utilisation des composés de formule (IV) selon la revendication 5, à titre de composés intermédiaires pour la synthèse des cyano-4 pyridazines par l'un quelconque des procédés selon l'une des revendications 1 à 3.

7. Utilisation des cyano-4 pyridazines obtenues selon l'une des revendications 1 à 3, en combinaison avec un support pharmaceutiquement approprié, pour la préparation de compositions pharmaceutiques.

8. Utilisation des cyano-4 pyridazines obtenues selon l'une des revendications 1 à 3, en combinaison avec un support pharmaceutiquement approprié, pour la préparation de compositions pharmaceutiques ayant une action psychotrope.

9. Utilisation selon la revendication 8, pour la préparation de compositions pharmaceutiques, conditionnées sous forme de doses unitaires pour l'administration orale, contenant de 1 à 500 mg d'ingrédient actif.

**Claims**

for the contracting states BE CH DE FR GB IT LI LU NL SE

1. As new products, the 4-cyanopyridazines corresponding to the general formula:

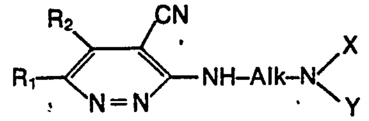

(I)

in which:

– one of the substituents $R_1$ and $R_2$ represents hydrogen and the other represents hydrogen; a $C_1$–$C_6$ alkyl group; a $C_3$–$C_7$ cycloalkyl group; a phenyl group; a phenyl group monosubstituted by a halogen atom, a trifluoromethyl group, a hydroxyl group, a $C_1$–$C_6$ alkoxy group, a $C_1$–$C_6$ alkyl group, a $C_1$–$C_6$ alkanoyloxy group, a cyano group, a $C_1$–$C_6$ alkylthio group, a $C_1$–$C_6$ alkylsulphinyl group, a $C_1$–$C_6$ alkylsulphonyl group or a sulphamyl group; a phenyl group disubstituted by one of the abovementioned substituents for the monosubstituted phenyl group and the other being a chlorine or fluorine atom or a methoxy group; a naphth-1-yl group; a naphth-2-yl group; a thien-2-yl group; a thien-3-yl group; or an indol-3-yl group;

– Alk represents an ethylene group, a 1,2-propylene group or a 1,3-propylene group;

– X denotes hydrogen; and

– Y represents hydrogen or a β-hydroxyethyl group, or alternatively

the group 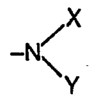

represents a morpholin-4-yl or 3-oxomorpholin-4-yl group, and also their pharmaceutically acceptable salts.

2. 3-(2-morpholinoethylamino)-4-cyano 5,6-diphenylpyridazine or one of its pharmaceutically acceptable salts.

3. 3-(2-morpholinoethylamino)-4-cyano 6-(4-nitrophenyl)pyridazine or one of its pharmaceutically acceptable salts.

4. Process for the preparation of the 4-cyanopyridazines according to claim 1, characterized in that it consists in:

a) treating an appropriately substituted 4-ethoxycarbonylpyridazone corresponding to the formula

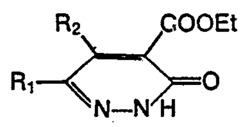
(II)

with aqueous ammonia to give the 4-carboxamide pyridazone of the formula (III):

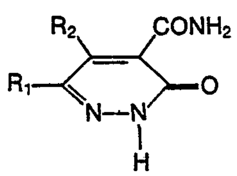
(III)

b) treating the 4-carboxamide pyridazone with phosphorus oxychloride to give the 3-chloro-4-cyanopyridazine of the formula IV:

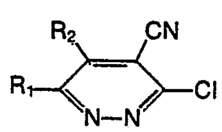
(IV)

and

c) reacting the said 3-chloro-4-cyanopyridazine with the amine of the formula

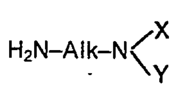

in which Alk is as defined in claim 1 and the group

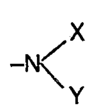

represents either $-NH_2$, or the 4-morpholino group, or the $-NHCH_2CH_2OH$ group, isolating the compound of formula (I) thus obtained in the form of free base or in the form of salt; optionally treating the resulting compound of formula (I) in which the

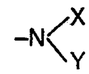

group is $-NHCH_2CH_2OH$ with chloroacetyl chloride in basic solution, and cyclizing the compound thus obtained by treatment with sodium methylate to obtain the product of formula (I) in which the radical

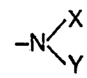

represents a 3-oxo morpholinyl group; said product being isolated in the form of base or in the form of salt; the radicals $R_1$, $R_2$ in the formulae II to IV above being as defined in claim 1.

5. Process for the preparation of the 4-cyanopyridazine according to claim 2, characterized in that it consists in:

a) treating the 4-ethoxycarbonylpyridazone corresponding to the formula

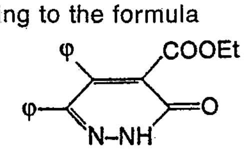
. (II)

with aqueous ammonia to give the 4-carboxamide pyridazone of the formula (III)

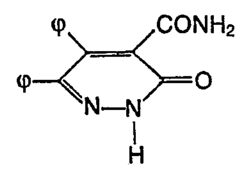

b) treating the 4-carboxamide pyridazone with phosphorus oxychloride to give the 3-chloro-4-cyanopyridazine of the formula (IV):

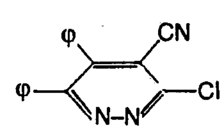

and

c) reacting the said 3-chloro-4-cyanopyridazine with the amine of the formula

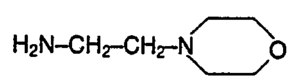

to form the expected compound, this compound being isolated in the form of free base or in the form of salt.

6. Process for the preparation of the 4-cyanopyridazine according to claim 3, characterized in that it consists in:

a) treating the 4-ethoxycarbonylpyridazone corresponding to the formula

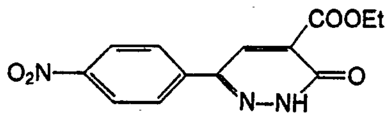

(II)

with aqueous ammonia to give the 4-carboxamide pyridazone of the formula

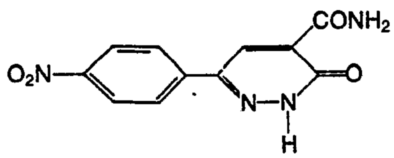

(III)

b) treating the pyridazone-4-carboxamide with phosphorus oxychloride to give the 3-chloro-4-cyanopyridazine of the formula (IV):

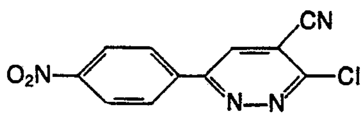

(IV)

and

c) reacting the said 3-chloro-4-cyanopyridazine with the amine of the formula

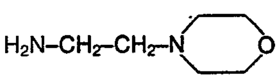 ,

to form the expected compound, this compound being isolated in the form of free base or in the form of salt.

7. Process according to any one of claims 4 to 6, characterized in that the 4-ethoxycarbonyl pyridazones of the formula II are prepared by reacting an α-halogenoketone of the formula

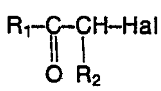

in which Hal is a chlorine or bromine atom, $R_1$ and $R_2$ being such as defined in claim 1, or $R_1$ and $R_2$ represent a phenyl group or $R_1$ represents a 4-nitrophenyl group whereas $R_2$ represents hydrogen; with ethyl malonate to form the substituted malonate:

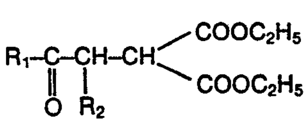

which is then cyclised by reaction with hydrazine to give the compound of the formula:

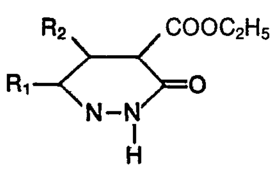

(C)

which is then dehydrogenated to form the desired compound of the formula II.

8. As intermediates for the synthesis of the 4-cyanopyridazines by any process according to any one of claims 4 to 6, the compounds of the formula IV below:

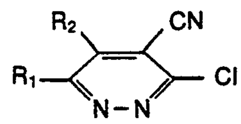

(IV)

in which $R_1$ and $R_2$ being such as defined in claim 1, represent a phenyl group or $R_1$ represents a 4-nitrophenyl group whereas $R_2$ represents hydrogen, provided that at least one of the groups $R_1$ or $R_2$ is other than hydrogen or the methyl group.

9. Process for the preparation of the 4-cyanopyridazines according to claim 8, characterized in that it consists in:

a) treating an appropriately substituted 4-ethoxycarbonylpyridazone corresponding to the formula

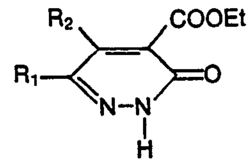

with aqueous ammonia to give the 4-carboxamide pyridazone of the formula (III)

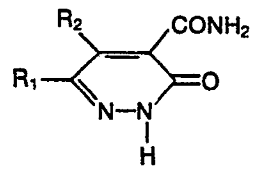

b) treating the 4-carboxamide pyridazone with phosphorus oxychloride, the radicals $R_1$, $R_2$ being as defined in claim 8.

10. Pharmaceutical compositions, characterized in that they contain as the active ingredient, at least a 4-cyanopyridazine according to any one of claims 1 to 3, in combination with a suitable pharmaceutical carrier.

11. Pharmaceutical compositions having a psychotropic action characterized in that they contain as active ingredient, at least a 4-cyanopyridazine according to any one of claims 1 to 3, in combination with a suitable pharmaceutical carrier.

12. Pharmaceutical compositions according to claim 11, characterized in that they are packaged in the form of unit doses for oral administration, containing 1 to 500 mg of active ingredient.

**Claims**

for the contracting state AT

1. Process for the preparation of the 4-cyanopyridazines corresponding to the general formula:

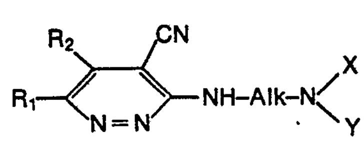

(I)

in which:

– one of the substituents $R_1$ and $R_2$ represents hydrogen and the other represents hydrogen; a

$C_1$–$C_6$ alkyl group; a $C_3$–$C_7$ cycloalkyl group; a phenyl group; a phenyl group monosubstituted by a halogen atom, a trifluoromethyl group, a hydroxyl group, a $C_1$–$C_6$ alkoxy group, a $C_1$–$C_6$ alkyl group, a $C_1$–$C_6$ alkanoyloxy group, a cyano group, a $C_1$–$C_6$ alkylthio group, a $C_1$–$C_6$ alkylsulphinyl group, a $C_1$–$C_6$ alkylsulphonyl group or a sulphamyl group; a phenyl group disubstituted by one of the abovementioned substituents for the monosubstituted group, the other substituent being a chlorine or fluorine atom or a methoxy group; a naphth-1-yl group; a naphth-2-yl group; a thien-2-yl group; a thien-3-yl group; or an indol-3-yl group;

– Alk represents an ethylene group, a 1,2-propylene group or a 1,3-propylene group;

– X denotes hydrogen; and

– Y represents hydrogen or a β-hydroxyethyl group, or alternatively

the group 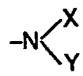

represents a morpholin-4-yl or 3-oxomorpholin-4-yl group, and also their pharmaceutically acceptable salts, characterised in that it consists in:

a) treating an appropriately substituted 4-ethoxycarbonylpyridazone corresponding to the formula

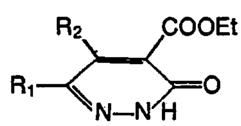

(II)

with aqueous ammonia to give the 4-carboxamide pyridazone of the formula (III)

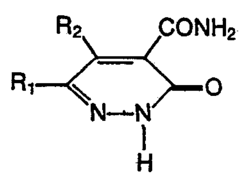

(III)

b) treating the 4-carboxamide pyridazone with phosphorus oxychloride to give the 3-chloro-4-cyano-pyridazine of the formula IV

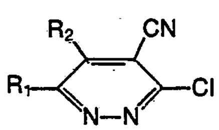

(IV)

and

c) reacting the said 3-chloro-4-cyanopyridazine with the amine of the formula

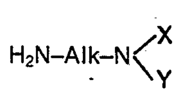 ,

in which Alk is as defined above and the group

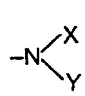

represents either $-NH_2$, or the 4-morpholino group, or the $-NHCH_2CH_2OH$ group, isolating the compound of formula (I) thus obtained in the form of free base or in the form of salt; optionally treating the resulting compound of formula (I) in which the

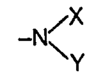

group is $-NHCH_2CH_2OH$ with chloroacetyl chloride in basic solution, and cyclizing the compound thus obtained by treatment with sodium methylate to obtain the product of formula (I) in which the radical

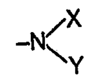

represents a 3-oxo morpholinyl group; said product being isolated in the form of base or in the form of salt; the radicals $R_1$, $R_2$ in the formulae II to IV above being as defined above.

2. Process for the preparation of 3-(2-morpholinoethylamino)-4-cyano-5,6-diphenyl pyridazine, or one of its pharmaceutically acceptable salts, characterised in that it consists in:

a) treating the 4-ethoxycarbonylpyridazone corresponding to the formula

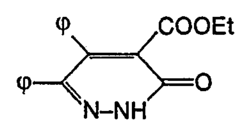

. (II)

with aqueous ammonia to give the 4-carboxamide pyridazone of the formula (III)

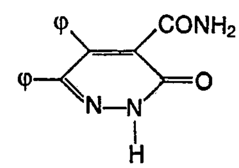

b) treating the 4-carboxamide pyridazone with phosphorus oxychloride to give the 3-chloro-4-cyanopyridazine of the formule IV

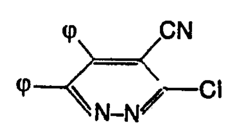

and

c) reacting the said 3-chloro-4-cyanopyridazine with the amine of the formula

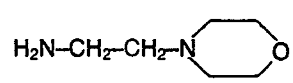

to form the expected compound, this compound being isolated in the form of free base or salt.

3. Process for the preparation of the 3-(2-morpholinoethylamino)-4-cyano-6-(4-nitrophenyl)pyridazine, or one of its pharmaceutically acceptable salts, characterised in that it consists in:

a) treating the 4-ethoxycarbonylpyridazone corresponding to the formula:

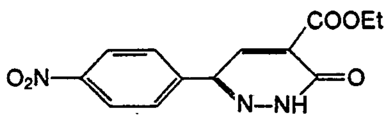
(II)

with aqueous ammonia to give the 4-carboxamide pyridazone of the formula

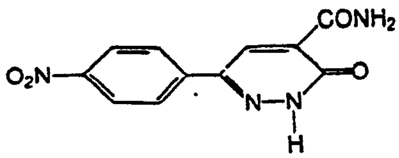
(III)

b) treating the 4-carboxamide pyridazone with phosphorus oxychloride to give the 3-chloro-4-cyanopyridazine of the formula (IV):

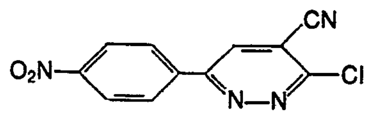
(IV)

and

c) reacting the said 3-chloro-4-cyanopyridazine with the amine of the formula

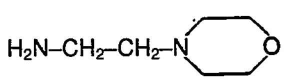

to form the expected compound, this compound being isolated in the form of free base or in the form of salt.

4. Process according to any one of claims 1 to 3, characterised in that the 4-ethoxycarbonyl pyridazones of the formula (II) are prepared from an α-halogenoketone of the formula:

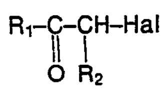

in which Hal is a chlorine or bromine atom, $R_1$ and $R_2$ being such as defined in claim 1, or $R_1$ and $R_2$ represent a phenyl group or $R_1$ represents a 4-nitrophenyl group whereas $R_2$ represents hydrogen; with ethyl malonate to form the substituted malonate:

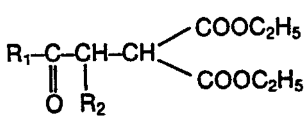

wich is then cyclised by reaction with hydrazine to give the compound of the formula:

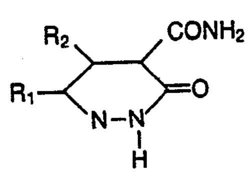
(C)

which is then dehydrogenated to form the desired compound of the formula II.

5. Process for the preparation of compounds of formula (IV)

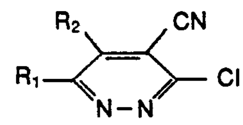
(IV)

in which $R_1$ and $R_2$ being such as defined in claim 1, or $R_1$ and $R_2$ represent a phenyl group or $R_1$ represents a 4-nitrophenyl group whereas $R_2$ represents hydrogen, provided that at least one of the groups $R_1$ or $R_2$ is other than hydrogen or the methyl group, characterised in that it consists in:

a) treating an appropriately substituted 4-ethoxycarbonylpyridazone corresponding to the formula

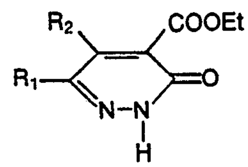

with aqueous ammonia to give the 4-carboxamide pyridazone of the formula (III):

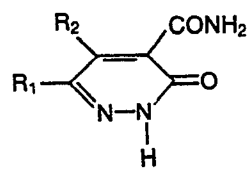

b) treating the 4-carboxamide pyridazone with phosphorus oxychloride; the radicals $R_1$ and $R_2$ being such as defined above.

6. Use of the compounds of formula IV according to claim 5, as intermediates for the synthesis of the 4-cyanopyridazines by any one of the processes according to any one of claims 1 to 3.

7. Use of the 4-cyanopyridazines obtained according to any one of claims 1 to 3, in combination with a suitable pharmaceutical carrier for the preparation of pharmaceutical compositions.

8. Use of 4-cyanopyridazines according to any one of claims 1 to 3, in combination with a suitable pharmaceutical carrier, for the preparation of pharmaceutical compositions having a psychotropic action.

9. Use according to claim 8, for the preparation of pharmaceutical compositions, packaged in the form of unit doses for oral administration, containing 1 to 500 mg of active ingredient.

**Patentansprüche**

für die Vertragsstaaten BE, CH, DE, FR, GB, LI, IT, LU, NL, SE:

1. Als neue Verbindungen 4-Cyano-pyridazine der allgemeinen Formel

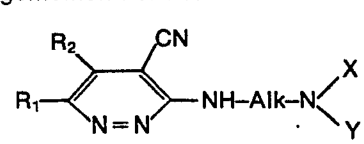
(I)

worin:

– einer der Substituenten $R_1$ oder $R_2$ Wasserstoff und der andere Wasserstoff, einen $C_1$–$C_6$-Alkyl-

gruppe, eine $C_3$–$C_7$-Cycloalkylgruppe, eine Phenylgruppe, eine durch ein Halogenatom, eine Trifluormethylgruppe, eine Hydroxygruppe, eine $C_1$–$C_6$-Alkoxygruppe, eine $C_1$–$C_6$-Alkylgruppe, eine $C_1$–$C_6$-Alkanoyloxygruppe, eine Cyanogruppe, eine $C_1$–$C_6$-Alkylthiogruppe, eine $C_1$–$C_6$-Alkylsulfinylgruppe, eine $C_1$–$C_6$-Alkylsulfonylgruppe oder eine Sulfamylgruppe monosubstituierte Phenylgruppe, eine durch einen der oben für die monosubstituierte Phenylgruppe genannten Substituenten disubstituierte Phenylgruppe, wobei der andere ein Chlor- oder Fluoratom oder eine Methoxygruppe ist; eine 1-Naphthylgruppe, eine 2-Naphthylgruppe, eine 2-Thienylgruppe, eine 3-Thienylgruppe eine 3-Indolylgruppe darstellt;

– Alk für eine Ethylengruppe, eine 1,2-Propylengruppe oder eine 1,3-Propylengruppe steht;

– X Wasserstoff bezeichnet;

– Y Wasserstoff oder eine β-Hydroxyethylgruppe darstellt,

– die Gruppe

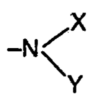

für eine 4-Morpholino- oder 3-Oxomorpholin-4-ylgruppe steht;

sowie ihre pharmazeutisch akzeptablen Salze.

2. 3-(2-Morpholino-ethylamino)-4-cyano-5,6-diphenylpyridazin oder eines seiner pharmazeutisch akzeptablen Salze.

3. 3-(2-Morpholino-ethylamino)-4-cyano-6-(4-nitrophenyl)-pyridazin oder eines seiner pharmazeutisch akzeptablen Salze.

4. Verfahren zur Herstellung der 4-Cyanopyridazine nach Anspruch 1, dadurch gekennzeichnet, dass es darin besteht, dass:

a) ein zweckmässig substituiertes 4-Ethoxycarbonyl-pyridazon der Formel

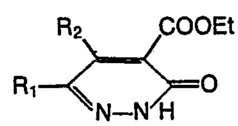 (II)

mit Ammoniak derart behandelt wird, dass 4-Carboxamid-pyridazon der Formel (III)

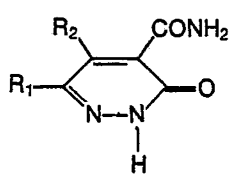 (III)

erhalten wird;

b) 4-Carboxamid-pyridazon mit Phosphoroxichlorid behandelt wird, um 3-Chlor-4-cyano-pyridazin der Formel IV

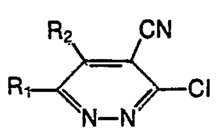 (IV)

zu erhalten;

c) 3-Chlor-4-cyano-pyridazin mit einem Amin der Formel

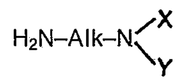 ,

worin Alk wie im Anspruch 1 definiert ist und die Gruppe

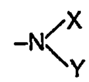

für $-NH_2$ oder eine 4-Morpholinogruppe oder die Gruppe $-NHCH_2CH_2OH$ steht, reagieren gelassen wird, die so erhaltene Verbindung der Formel (I) in Form der freien Base oder in Salzform isoliert wird; die erhaltene Verbindung

der Formel (I), worin die Gruppe 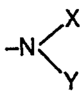 für

$-NHCH_2CH_2OH$ steht, gegebenenfalls mit Chloracetylchlorid in basischer Lösung behandelt wird und die so erhaltene Verbindung durch Behandlung mit Natriummethylat zyklisiert wird, um das Produkt der Formel (I) zu erhalten, worin der Rest

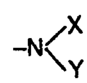

eine 3-Oxomorpholinylgruppe darstellt; wobei das Produkt in Form der Base oder in Salzform isoliert wird; wobei die Reste $R_1$, $R_2$ in den obigen Formeln II bis IV wie in Anspruch 1 definiert sind.

5. Verfahren zur Herstellung des 4-Cyano-pyridazins nach Anspruch 2, dadurch gekennzeichnet, dass es darin besteht, dass:

a) 4-Ethoxycarbonyl-pyridazon der Formel

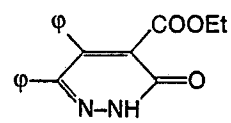 (II)

mit Ammoniak derart behandelt wird, dass 4-Carboxamid-pyridazon der Formel (III)

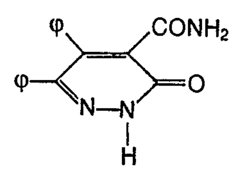

erhalten wird;

b) 4-Carboxamid-pyridazon mit Phosphoroxichlorid behandelt wird, um das 3-Chlor-4-cyano-pyridazin der Formel (IV)

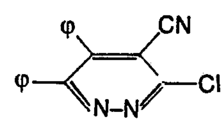

zu erhalten;

c) diese mit dem Amin der Formel

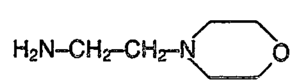

reagieren gelassen wird, um die erwartete Verbindung zu bilden, wobei letztere in Form der freien Base oder in Salzform isoliert wird.

6. Verfahren zur Herstellung des 4-Cyano-pyridazins nach Anspruch 3, dadurch gekennzeichnet, dass es darin besteht, dass:

a) 4-Ethoxycarbonyl-pyridazon der Formel

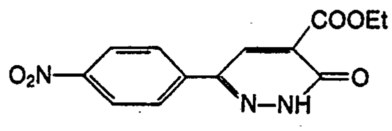

(II)

mit Ammoniak derart behandelt wird, dass 4-Carboxamid-pyridazon der Formel

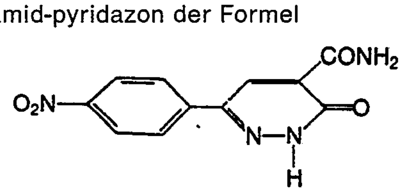

(III)

erhalten wird;

b) 4-Carboxamid-pyridazon mit Phosphoroxichlorid behandelt wird, um 3-Chlor-4-cyano-pyridazin der Formel (IV)

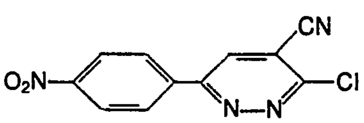

(IV)

zu erhalten;

c) dieses mit dem Amin der Formel

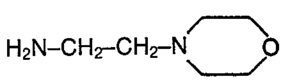

reagieren gelassen wird, um die erwartete Verbindung zu erhalten, wobei letztere in Form der freien Base oder in Salzform isoliert wird.

7. Verfahren nach einem der Ansprüche 4 bis 6, dadurch gekennzeichnet, dass die 4-Ethoxycarbonyl-pyridazone der Formel (II) hergestellt werden aus einem α-Haloketon der Formel

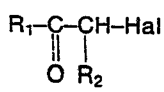

worin Hal ein Chlor- oder Bromatom ist, $R_1$ und $R_2$ wie in Anspruch 1 definiert sind oder $R_1$ und $R_2$ eine Phenylgruppe darstellen, oder $R_1$ eine 4-Nitro-phenylgruppe bedeutet, während $R_2$ für Wasserstoff steht; mit Ethylmalonat, um das substituierte Malonat

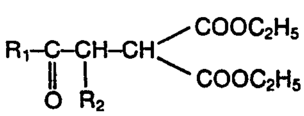

zu bilden, welches danach durch Einwirken von Hydrazin zyklisiert wird, um die Verbindung der Formel

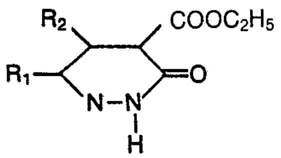

(C)

zu erhalten, die dann zur Bildung der gewünschten Verbindung der Formel II dehydriert wird.

8. Als intermediäre Verbindungen für die Synthese der 4-Cyano-pyridazine mittels einem der Verfahren nach einem der Ansprüche 4 bis 6 die Verbindungen der nachstehenden Formel IV

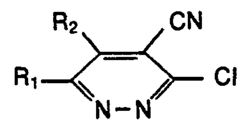

(IV)

worin $R_1$ und $R_2$ wie in Anspruch 1 definiert sind oder $R_1$ und $R_2$ eine Phenylgruppe darstellen, oder $R_1$ eine 4-Nitrophenylgruppe bedeutet, während $R_2$ für Wasserstoff steht, mit der Massgabe, dass zumindest eine der Gruppen $R_1$ oder $R_2$ anders als Wasserstoff oder eine Methylgruppe ist.

9. Verfahren zur Herstellung der 4-Cyano-pyridazine nach Anspruch 8, dadurch gekennzeichnet, dass es darin besteht, dass:

a) ein zweckmässig substituiertes 4-Ethoxycarbonyl-pyridazon der Formel

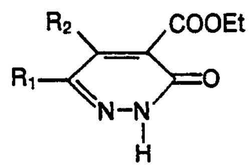

mit Ammoniak derart behandelt wird, dass 4-Carboxamid-pyridazon der Formel (III)

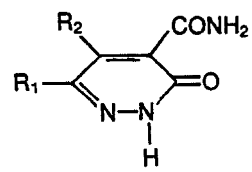

erhalten wird;

b) 4-Carboxamid-pyridazon mit Phosphoroxichlorid behandelt wird, wobei die Reste $R_1$ und $R_2$ wie in Anspruch 8 definiert sind.

10. Pharmazeutische Zusammensetzungen, dadurch gekennzeichnet, dass sie als aktives Ingrediens mindestens ein 4-Cyano-pyridazin nach einem der Ansprüche 1 bis 3 in Kombination mit einem geeigneten pharmazeutischen Träger enthalten.

11. Pharmazeutische Zusammensetzungen mit psychotroper Wirkung, dadurch gekennzeichnet, dass sie als aktives Ingrediens mindestens ein 4-Cyano-pyridazin nach einem der Ansprüche 1 bis 3 in Kombination mit einem geeigneten pharmazeutischen Träger enthalten.

12. Pharmazeutische Zusammensetzungen nach Anspruch 11, dadurch gekennzeichnet, dass sie in Dosiseinheitsform für orale Verabreichung, enthaltend 1 bis 500 mg aktives Ingrediens, konditioniert sind.

**Patentansprüche**
für den Vertragsstaat AT:

1. Verfahren zur Herstellung von 4-Cyano-pyridazinen der allgemeinen Formel

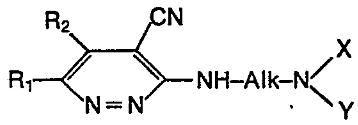

(I)

worin:

– einer der Substituenten $R_1$ oder $R_2$ Wasserstoff und der andere Wasserstoff, eine $C_1$–$C_6$-Alkylgruppe, eine $C_3$–$C_7$-Cycloalkylgruppe, eine Phenylgruppe, eine durch ein Halogenatom, eine Trifluormethylgruppe, eine Hydroxygruppe, eine $C_1$–$C_6$-Alkoxygruppe, eine $C_1$–$C_6$-Alkylgruppe, eine $C_1$–$C_6$-Alkanoyloxygruppe, eine Cyanogruppe, eine $C_1$–$C_6$-Alkylthiogruppe, eine $C_1$–$C_6$-Alkylsulfinylgruppe, eine $C_1$–$C_6$-Alkylsulfonylgruppe oder eine Sulfamylgruppe monosubstituierte Phenylgruppe, eine durch einen der oben für die monosubstituierte Phenylgruppe genannten Substituenten disubstituierte Phenylgruppe, wobei der andere ein Chlor- oder Fluoratom oder eine Methoxygruppe ist; eine 1-Naphthylgruppe, eine 2-Naphthylgruppe, eine 2-Thienylgruppe, eine 3-Thienylgruppe eine 3-Indolylgruppe darstellt;

– Alk für eine Ethylengruppe, eine 1,2-Propylengruppe oder eine 1,3-Propylengruppe steht;

– X Wasserstoff bezeichnet;

– Y Wasserstoff oder eine β-Hydroxyethylgruppe darstellt,

– oder aber die Gruppe

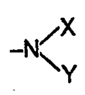

für eine 4-Morpholino- oder 3-Oxomorpholin-4-ylgruppe steht;

sowie ihrer pharmazeutisch akzeptablen Salze, dadurch gekennzeichnet, dass es darin besteht, dass

a) ein zweckmässig substituiertes 4-Ethoxycarbonyl-pyridazon der Formel

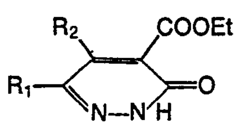

(II)

mit Ammoniak derart behandelt wird, dass 4-Carboxamid-pyridazon der Formel (III)

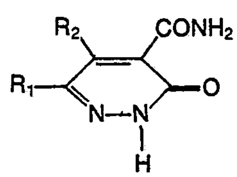

(III)

erhalten wird;

b) 4-Carboxamid-pyridazon mit Phosphoroxichlorid behandelt wird, um 3-Chlor-4-cyano-pyridazin der Formel IV

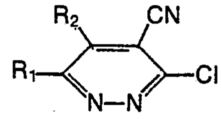

(IV)

zu erhalten;

c) 3-Chlor-4-cyano-pyridazin mit einem Amin der Formel

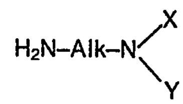 ,

worin Alk wie im Anspruch 1 definiert ist und die Gruppe

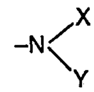

für $-NH_2$ oder eine 4-Morpholinogruppe oder die Gruppe $-NHCH_2CH_2OH$ steht, reagieren gelassen wird, die so erhaltene Verbindung der Formel (I) in Form der freien Base oder in Salzform isoliert wird; die erhaltene Verbindung der Formel (I), worin die Gruppe

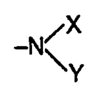

für $-NHCH_2CH_2OH$ steht, gegebenenfalls mit Chloracetylchlorid in basischer Lösung behandelt wird und die so erhaltene Verbindung durch Behandlung mit Natriummethylat zyklisiert wird, um das Produkt der Formel (I) zu erhalten, worin der Rest

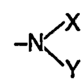

eine 3-Oxomorpholinylgruppe darstellt; wobei das Produkt in Form der Base oder in Salzform isoliert wird; wobei die Reste $R_1$, $R_2$ in den obigen Formeln II bis IV wie oben definiert sind.

2. Verfahren zur Herstellung von 3-(2-Morpholino-ethylamino)-4-cyano-5,6-diphenyl-pyridazin oder eines seiner pharmazeutisch akzeptablen Salze, dadurch gekennzeichnet, dass es darin besteht, dass

a) 4-Ethoxycarbonyl-pyridazon der Formel

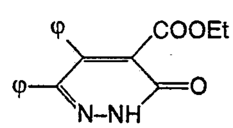

(II)

mit Ammoniak derart behandelt wird, dass 4-Carboxamid-pyridazon der Formel (III)

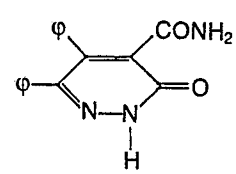

erhalten wird;

b) 4-Carboxamid-pyridazon mit Phosphoroxichlorid behandelt wird, um das 3-Chlor-4-cyano-pyridazin der Formel (IV)

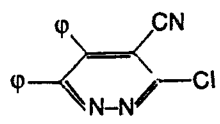

zu erhalten;

c) diese mit dem Amin der Formel

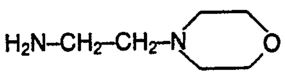

reagieren gelassen wird, um die erwartete Verbindung zu bilden, wobei letztere in Form der freien Base oder in Salzform isoliert wird.

3. Verfahren zur Herstellung von 3-(2-Morpholino-ethylamino)-4-cyano-6-(4-nitrophenyl)-pyridazin oder eines seiner pharmazeutisch akzeptablen Salze, dadurch gekennzeichnet, dass es darin besteht, dass

a) 4-Ethoxycarbonyl-pyridazon der Formel

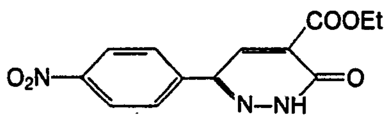 (II)

mit Ammoniak derart behandelt wird, dass 4-Carboxamid-pyridazon der Formel

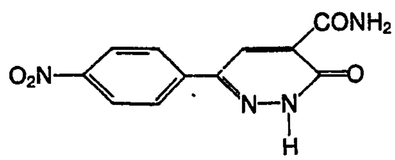 (III)

erhalten wird;

b) 4-Carboxamid-pyridazon mit Phosphoroxichlorid behandelt wird, um 3-Chlor-4-cyano-pyridazin der Formel (IV)

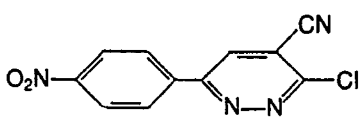 (IV)

zu erhalten;

c) dieses mit dem Amin der Formel

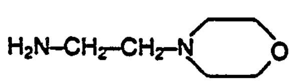

reagieren gelassen wird, um die erwartete Verbindung zu erhalten, wobei letztere in Form der freien Base oder in Salzform isoliert wird.

4. Verfahren nach einem der Ansprüche 1 bis 3, dadurch gekennzeichnet, dass die 4-Ethoxycarbonyl-pyridazone der Formel (II) hergestellt werden aus einem α-Haloketon der Formel

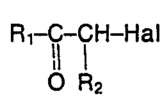

worin Hal ein Chlor- oder Bromatom ist, $R_1$ und $R_2$ wie in Anspruch 1 definiert sind oder $R_1$ und $R_2$ eine Phenylgruppe darstellen, oder $R_1$ eine 4-Nitro-phenylgruppe bedeutet, während $R_2$ für Wasserstoff steht; mit Ethylmalonat, um das substituierte Malonat

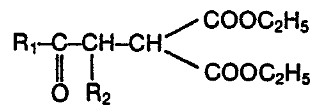

zu bilden, welches danach durch Einwirken von Hydrazin zyklisiert wird, um die Verbindung der Formel

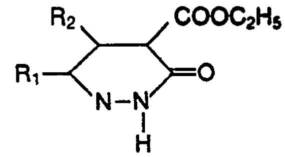 (C)

zu erhalten, die dann zur Bildung der gewünschten Verbindung der Formel II dehydriert wird.

5. Verfahren zur Herstellung der Verbindungen der nachstehenden Formel (IV)

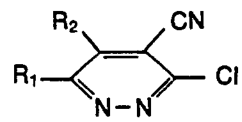 (IV)

worin $R_1$ und $R_2$ wie in Anspruch 1 definiert sind oder $R_1$ und $R_2$ eine Phenylgruppe darstellen, oder $R_1$ eine 4-Nitrophenylgruppe bedeutet, während $R_2$ für Wasserstoff steht, mit der Massgabe, dass zumindest eine der Gruppen $R_1$ oder $R_2$ anders als Wasserstoff oder eine Methylgruppe ist, dadurch gekennzeichnet, dass es darin besteht, dass:

a) ein zweckmässig substituiertes 4-Ethoxycarbonyl-pyridazon der Formel

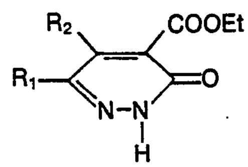

mit Ammoniak derart behandelt wird, dass 4-Carboxamid-pyridazon der Formel (III)

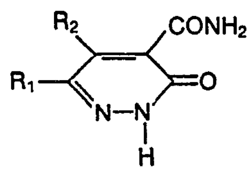

erhalten wird;

b) 4-Carboxamid-pyridazon mit Phosphoroxichlorid behandelt wird; wobei die Reste $R_1$ und $R_2$ wie oben definiert sind.

6. Verwendung der Verbindungen der Formel (IV) nach Anspruch 5 als intermediäre Verbindungen für die Synthese der 4-Cyano-pyridazine mittels eines der Verfahren nach einem der Ansprüche 1 bis 3.

7. Verwendung der nach einem der Ansprüche 1 bis 3 erhaltenen 4-Cyano-pyridazine in Kombination mit einem pharmazeutisch akzeptablen Träger zur Herstellung von pharmazeutischen Zusammensetzungen.

8. Verwendung der nach einem der Ansprüche 1 bis 3 erhaltenen 4-Cyano-pyridazine in Kombination mit einem pharmazeutisch akzeptablen Träger zur Herstellung von pharmazeutischen Zusammensetzungen mit psychotroper Wirkung.

9. Verwendung nach Anspruch 8 zur Herstellung von pharmazeutischen Zusammensetzungen, die in Dosiseinheitsform für orale Verabreichung, enthaltend 1 bis 500 mg aktives Ingrediens, konditioniert sind.